# FASCICULE DE BREVET EUROPEEN

(11) **EP 2 877 432 B1**
(45) Date de publication et mention de la délivrance du brevet: **21.03.2018**
(21) Numéro de dépôt: 13774796.0
(22) Date de dépôt: 25.07.2013
(51) Int. Cl.: C02F 9/00, C12M 1/34, C12M 1/107, C12M 1/00, C02F 3/28, C02F 3/30, C02F 3/12, C02F 3/02, C02F 3/00, C02F 11/04, C02F 11/12

(54) **PROCÉDÉ DE RÉDUCTION DE LA PRODUCTION DE BOUES DE STATIONS D'ÉPURATION D'EAUX USÉES URBAINES OU INDUSTRIELLES, ET INSTALLATION POUR SA MISE EN OEUVRE**
VERFAHREN ZUR VERMINDERUNG DER PRODUKTION VON SCHLAMM DURCH STÄDTISCHE ODER INDUSTRIELLE ABWASSERREINIGUNGSANLAGEN UND AUSRÜSTUNG ZU SEINER DURCHFÜHRUNG
PROCESS FOR REDUCING THE PRODUCTION OF SLUDGE BY MUNICIPAL OR INDUSTRIAL WASTEWATER PURIFICATION PLANTS, AND EQUIPMENT FOR THE IMPLEMENTATION THEREOF

(30) Priorité: 26.07.2012 FR 1257253
(43) Date de publication de la demande: 03.06.2015
(73) Titulaire: Degrémont, 92040 Paris La Défense (FR)
(72) Inventeur: PARDO, Pierre, Emmanuel, F-91400 Orsay (FR)
(74) Mandataire: Cabinet Armengaud Aîné
(86) Numéro de dépôt international: PCT/IB2013/056107
(87) Numéro de publication internationale: WO 2014/016797

(56) Documents cités:
- EP-A1- 0 620 273
- WO-A2-2006/029971
- WO-A2-2006/124781
- DE-A1- 10 107 712
- DE-A1- 19 527 784
- JP-A- 2007 021 413
- US-A- 6 015 496
- US-A1- 2006 256 645
- US-A1- 2010 213 121

## Description

La présente invention concerne un procédé de réduction de la production de boues de stations d'épuration d'eaux usées urbaines ou industrielles, du genre de ceux qui comprennent une étape de digestion anaérobie mésophile ou thermophile, ou associant ces deux modes de fonctionnement, d'un flux de boues à traiter, et au moins une étape de traitement aérobie de solubilisation biologique des boues.

Un procédé de ce genre est connu, notamment d'après FR 2 849 019. Selon ce procédé antérieur, on applique à la boue digérée un traitement aérobie avec stress enzymatique thermophile, notamment de 60 à 70 °C, durant 24 à 72 h, ce qui permet d'obtenir, en fonction des conditions du stress, des solubilisations de 20 à 40 % de la matière organique et de 5 à 25 % de la matière minérale contenues dans les boues issues du procédé de digestion anaérobie, et en faisant retourner une partie de cette boue au digesteur. Ce procédé présente toutefois plusieurs inconvénients, dont ceux exposés ci-après :
- Si on veut pousser le traitement, il faut recirculer une grande quantité de liquide, ce qui surdimensionne le réacteur affecté à la digestion, laquelle est basée en particulier sur un temps de séjour hydraulique.
- Le stress enzymatique thermophile transforme l'azote organique en nitrates. Ces nitrates sont un polluant fort pour la digestion. Il y a donc un risque élevé de pollution si on veut pousser le traitement.
- Enfin si on place l'étape de stress enzymatique thermophile en amont de la digestion, la production de biogaz n'est pas augmentée significativement, seule la réduction de la production de boues a lieu.

En conséquence, la présente invention a pour but, surtout, de fournir un procédé de réduction de la production de boues de stations d'épuration d' eaux usées urbaines ou industrielles, par voie anaérobie, qui ne présente plus, ou à un degré moindre, les inconvénients rappelés ci-dessus. En particulier, il est souhaitable de limiter ou de réduire les dimensions du réacteur nécessaire à la digestion, tout en augmentant la production de biogaz.

Selon l'invention, le procédé de réduction de la production de boues de stations d'épuration d'eaux usées urbaines ou industrielles, comprend une étape de digestion anaérobie mésophile ou thermophile, ou associant ces deux modes de fonctionnement, d'un flux de boues à traiter, et au moins une étape de traitement aérobie de solubilisation biologique, et est caractérisé en ce qu'il comporte en amont de l'étape de digestion anaérobie :
- une étape de déshydratation des boues à traiter,
- suivie d'une étape de mélange des boues déshydratées avec une fraction recirculée de boues plus liquides provenant d'un recyclage de la digestion, et/ou de l'étape de traitement aérobie, et/ou de centrats et/ou de boues provenant d'une déshydratation finale des boues traitées, le taux de recirculation étant choisi pour que le mélange présente une siccité adaptée à la digestion, ce mélange étant ensuite dirigé vers la digestion.

De préférence, le taux de recirculation est également choisi pour que les concentrations en nitrates du mélange soient adaptées à la digestion.

Avantageusement, la siccité des boues en sortie de la première étape de déshydratation est comprise entre 20 et 30% de matières sèches en masse, et le taux de recirculation est choisi pour que la siccité des boues en sortie du mélange et à l'entrée de la digestion soit inférieure à 10% de matières sèches en masse, de préférence d'environ 6%. L'étape de traitement aérobie de solubilisation biologique est située en aval de la digestion anaérobie, en particulier sur une boucle de recirculation de l'étape de digestion anaérobie.

En ce qui concerne l'étape aérobie, on peut notamment mettre en oeuvre la technique décrite dans EP-A-924 168 ou EP-A-1 008 558. On peut aussi simplement mettre en place un bassin aéré sans chaleur mais la réaction globale est moins importante.

L'aération de la boue digérée permet de faire intervenir d'autres bactéries qui vont « couper » les molécules avec des liaisons C-O. Ces liaisons seront ensuite utilisées lors de la digestion pour continuer de dégrader la matière organique. L'étape aérobie permet donc de pousser la digestion.

L'étape de déshydratation en amont de la digestion permet de s'affranchir des problèmes de volume de digesteur.

Le procédé peut comporter une étape finale de déshydratation, en particulier par centrifugation.

Avantageusement, le procédé comporte une mesure de nitrates en entrée de la digestion, une mesure d'ammonium et de pH en sortie de la digestion, et une régulation de la recirculation à partir de ces mesures.

Le procédé peut également comporter une étape de dilacération/dessablage de la boue permettant de diminuer l'ensablement du digesteur, l'abrasion des composants et l'agglomération de filasses dans le circuit.

Une partie du biogaz produit lors de la digestion anaérobie des boues peut être utilisée comme source d'énergie pour chauffer ou maintenir en température un réacteur dans lequel s'effectue l'étape aérobie de solubilisation biologique.

Avantageusement, un échange de chaleur est effectué entre des boues sortant de l'étape aérobie de solubilisation biologique et des boues sortant de l'étape de digestion anaérobie et qui sont dirigées vers l'étape aérobie, pour que le procédé soit énergétiquement auto-équilibré et que la consommation énergétique soit réduite. L'échange de chaleur peut être du type indirect boues/eau, eau/boues, l'eau servant de fluide caloporteur pour l'échange entre les deux boues.

De préférence, une dilution est prévue sur une boucle de recirculation reliée à la sortie de l'étape aérobie, pour permettre de piloter les conditions de fonctionnement de la digestion.

Des flux de retour provenant de l'étape aérobie, ou d'une déshydratation finale, peuvent être pilotés, en particulier en fonction des conditions de fonctionnement de la digestion et de la production de biogaz et en fonction de la qualité du résidu solide (taux de matières volatiles et taux d'azote).

Etant donné que, selon l'invention, d'une part le procédé de réduction de production de boue est mis en oeuvre sur la filière de traitement des boues, et que d'autre part, comportant une étape de déshydratation au départ, il ne transporte que peu d'eau, il est indépendant de la filière de traitement de l'eau et ne conduit pas à des modifications significatives du fonctionnement de cette filière de traitement de l'eau.

Par ailleurs, la matière organique solubilisée lors de l'étape aérobie étant dégradée par voie anaérobie dans le procédé de l'invention, cette dégradation ne conduit pas à une surconsommation d'oxygène au niveau de la filière de traitement de l'eau et donc aucune augmentation de l'apport en oxygène sur cette ligne de traitement de l'eau.

La ligne de traitement des eaux devra par contre tenir compte de l'augmentation de nitrates en retour en tête.

Selon ces différents modes de mise en oeuvre, la présente invention permet d'augmenter de 20 à 50 % la cinétique de production de biogaz et permet d'augmenter de 30 à 100% le rendement de dégradation des matières organiques contenues dans la boue par rapport à un procédé conventionnel de digestion anaérobie des boues.

La présente invention permet d'améliorer de manière significative la réduction de la concentration en micro-organismes pathogènes dans la boue effluente et également une élimination partielle ou totale des micro-organismes filamenteux responsables du phénomène de moussage dans l'étape de digestion anaérobie.

Par ailleurs, la mise en oeuvre du procédé de l'invention permet une amélioration de la siccité des boues comprise entre 20 et 40 % par rapport aux procédés classiques de digestion anaérobie.

Le procédé selon l'invention est énergétiquement auto-équilibré et, selon ces différents modes de mise en oeuvre, une partie du biogaz produit lors de la digestion anaérobie des boues est utilisée comme source d'énergie pour chauffer ou maintenir en température (50 à 70 °C) le réacteur dans lequel s'effectue l'étape aérobie.

Selon la présente invention, et comme selon FR 2 849 019, le procédé peut comporter une étape de dé-phosphatation secondaire qui est mise en oeuvre dans le circuit de réduction de la production des boues, en complément de celle mise en oeuvre dans la ligne principale de traitement de l'eau. Dans ce cas, la pollution phosphorée est éliminée par précipitation chimique, par addition de sels métalliques et/ou de composés minéraux.

L'invention concerne également une installation pour la mise en oeuvre du procédé, cette installation comprenant un digesteur anaérobie de type mésophile ou thermophile, ou associant ces deux modes de fonctionnement, d'un flux de boues à traiter, et au moins un réacteur de traitement aérobie de solubilisation biologique des boues, et étant caractérisée en ce qu'elle comporte en amont du digesteur anaérobie :
- un dispositif de déshydratation des boues à traiter,
- suivi d'un mélangeur des boues déshydratées avec une fraction recirculée de boues plus liquides délivrées par une conduite de recyclage provenant du digesteur, et/ou par une conduite provenant du réacteur de traitement aérobie, et/ou par une conduite de centrats et/ou d'une fraction de boues solides provenant d'une déshydratation finale des boues traitées, le taux de recirculation étant choisi pour que le mélange présente une siccité adaptée à la digestion, et que les concentrations en nitrates soient adaptées à la digestion, le mélange étant ensuite dirigé vers la digestion.
Le réacteur de traitement aérobie est situé en aval du digesteur anaérobie, en particulier sur une boucle de recirculation du digesteur anaérobie.

L'installation peut comporter un dispositif de déshydratation finale, en particulier une centrifugeuse. L'installation peut également comporter un dispositif de dilacération/dessablage de la boue permettant de diminuer l'ensablement du digesteur, l'abrasion des composants et l'agglomération de filasses dans le circuit.

De préférence, l'installation comporte un dispositif de chauffage du réacteur aérobie, alimenté par une partie du biogaz produit par le digesteur anaérobie des boues.

L'installation peut comporter une sonde de nitrates sur l'entrée du digesteur et une sonde d'ammonium (ion ammonium NH4+) et de pH en sortie du digesteur. Ces sondes permettent de mieux piloter les recyclages.

L'installation peut comporter un échangeur de chaleur entre des boues sortant du réacteur aérobie et des boues sortant du digesteur anaérobie et qui sont dirigées vers le réacteur aérobie .

L'installation comporte avantageusement un dispositif de dilution prévu sur une boucle de recirculation provenant de la sortie du réacteur aérobie, pour permettre de piloter les conditions de fonctionnement de la digestion.

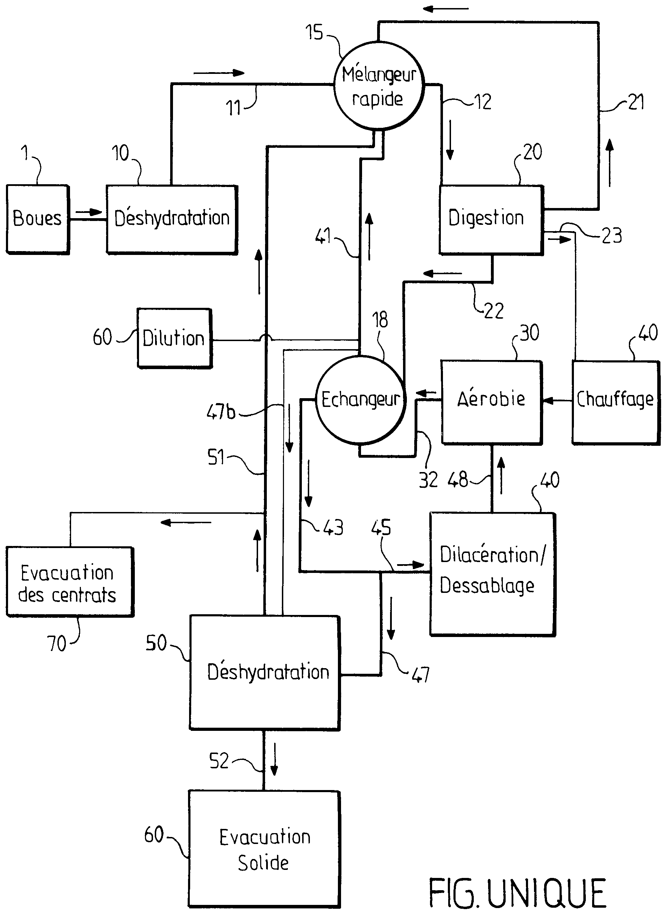

L'invention consiste, mises à part les dispositions exposées ci-dessus, en un certain nombre d'autres dispositions dont il sera plus explicitement question ci-après à propos d'un exemple de réalisation décrit avec référence au dessin annexé, mais qui n'est nullement limitatif. L'invention est définie par les revendications annexées.

Sur le dessin:
la figure unique est une représentation schématique d'une installation mettant en oeuvre le procédé de réduction de la production de boues, selon l'invention, avec couplage de la déshydratation, de l'étape de digestion anaérobie et de l'étape aérobie.

En se référant à la figure du dessin, on peut voir une installation de traitement des boues selon la présente invention, qui comporte, en amont, un dispositif de déshydratation 10 des boues 1 à traiter provenant d'une ligne de traitement d'eaux urbaines ou industrielles. Ce dispositif 10 peut être un filtre à bandes, une centrifugeuse, un filtre presse ou tout autre matériel de déshydratation permettant d'augmenter de manière significative la siccité des boues. Plus la siccité sera élevée, plus les gains de coûts de l'ensemble du procédé seront élevés. Typiquement, la siccité à atteindre sera de 20 à 30% de matières sèches en sortie du dispositif 10 de déshydratation. La déshydratation peut porter sur la totalité du flux de boues, ou sur une partie seulement, l'autre partie étant dérivée dans une conduite (non représentée) contournant le dispositif 10.

L'installation mettant en oeuvre le procédé de l'invention est avantageusement indépendante de la ligne de traitement des eaux usées ; les boues produites dans la ligne de traitement des eaux sont transportées ou transférées vers l'installation de l'invention.

La sortie du dispositif 10 de déshydratation est reliée par une conduite 11 à l'entrée d'un mélangeur rapide 15 qui permet de faire un mélange intime et homogène entre la boue déshydratée provenant de la déshydratation 10, celle provenant d'une autre déshydratation 50, et :
- de la boue diluée provenant d'un réacteur ou digesteur 20 assurant la digestion, cette boue étant mise en recirculation par une conduite 21 reliant une sortie du digesteur 20 et une entrée du mélangeur 15 ; le sens d'écoulement de la boue dans la conduite 21 est indiqué par une flèche ; cette recirculation 21 permet en particulier le maintien en température du digesteur 20 par le biais d'un échangeur, non représenté, en cas de besoin ;
- et/ou de la boue traitée par voie aérobie dans un réacteur aérobie 30, cette boue arrivant dans le mélangeur 15 par une conduite 41,
- et/ou le centrat, ou fraction liquide, recirculé par une conduite 51 aboutissant au mélangeur 15, et provenant de l'étape de déshydratation finale 50, notamment par centrifugation.

A noter que la conduite 51 peut en fonction des qualités de boues être définie pour transporter soit des centrats, soit de la boue déshydratée. En cas de nécessité de transporter les deux pour des besoins de régulation du procédé, deux canalisations 51 peuvent être mises en oeuvre (non représentées).

Des organes de mesure et de réglage de débit, notamment des électrovannes, non représentés, sont prévus sur les différentes conduites, notamment 21, 41, 51 pour régler les débits respectifs notamment en fonction du débit dans la conduite 11. Les sens d'écoulement dans les conduites sont indiqués par des flèches le long de ces conduites.

Des organes de mesure et régulation (non représentés) sont aussi prévus sur les différentes conduites, notamment une sonde de nitrates sur la conduite 12 et une sonde d'ammonium et de pH sur la conduite 22, qui fournissent des résultats de mesure à une régulation (non représentée) permettant de réguler les recirculations.

En fonction des différents flux, plusieurs configurations de mélange peuvent être prévues entre le débit de la conduite 11 d'une part, et les débits des conduites 21, 41, 51 d'autre part.

Les configurations de mélange sont prévues pour que la boue sortant du mélangeur 15 par la conduite 12, reliée à l'entrée du digesteur 20, présente une siccité adaptée à la digestion, en particulier une siccité égale ou inférieure à 6%, pour que la viscosité de la boue convienne à la digestion. Le mélangeur 15 peut être du type à mélange rapide (flash-mixing) avec de forts coefficients de mélange.

Le dispositif de digestion anaérobie des boues 20 est d'un type qui peut mettre en oeuvre un procédé de digestion anaérobie mésophile, thermophile ou associant ces deux modes de fonctionnement (avec ou sans étape d'acidogénèse).

Le réacteur aérobie 30, dimensionné pour des temps de séjour de 4 à 96 h, est maintenu à une température pouvant aller de la température ambiante 20°C à 75 °C, de préférence à une température de 60 à 70°C qui favorise les réactions. Ce réacteur peut être chauffé par un dispositif de chauffage 40, auquel est fourni, comme combustible, par une conduite 23, une partie du biogaz produit par le digesteur anaérobie 20 des boues. Tout autre moyen de récupération de l'énergie peut être prévu pour le chauffage, notamment un échangeur boues/boues 18 ou boues/eau , ou eau/boues.

Le réacteur 30 est aéré par insufflation d'air ou d'oxygène à l'aide de buses (non représentées) disposées dans le fond du réacteur. L'apport d'oxygène dans le réacteur 30 est au moins égal à celui nécessaire pour assurer la stoechiométrie de l'oxydation des matières à traiter ; de préférence l'apport d'oxygène est supérieur à la valeur stoechiométrique, et est qualifié de surstoechiométrique.

Une sortie du digesteur 20 est reliée à une conduite 22 qui dirige la boue digérée vers l'entrée du réacteur aérobie 30. De préférence, la conduite 22 est reliée à l'entrée d'un premier circuit d'un échangeur de chaleur 18, la sortie de ce premier circuit étant reliée à une conduite 43. L'autre circuit de l'échangeur 18 comporte une entrée reliée par une conduite 32 à la sortie du réacteur aérobie 30, et une sortie reliée par une conduite 41 au mélangeur 15.

Les boues sortant du réacteur 30 par la conduite 32 contribuent à échauffer, dans l'échangeur 18, les boues provenant par la conduite 22 du digesteur 20.

Ce bloc échangeur 18 peut être constitué d'un échangeur boues/eau, eau/boues où l'eau sert de média pour transférer la chaleur de la boue de la conduite 32 vers la boue de la conduite 22.

La conduite 43 se divise en deux branches formées par des conduites 45, 47. La conduite 45 se raccorde à l'entrée d'un dispositif de dilacération/dessablage 40 permettant de retirer le sable de la boue par exemple par traitement hydrocyclonique et d'éliminer des paquets de filasse. La sortie du dispositif 40 est reliée par une conduite 48 à l'entrée du réacteur aérobie 30.

Une boucle de recirculation 22, 43, 45, 48, 32, 41, 12 du digesteur anaérobie 20 est formée sur laquelle est installé le réacteur aérobie 30 entre les conduites 48 et 32. Le réacteur aérobie 30 est alimenté uniquement par des boues digérées provenant du digesteur 20.

La conduite 47 se raccorde à l'entrée d'un dispositif 50 de déshydratation des boues traitées, pouvant être un filtre à bande, une centrifugeuse, un filtre presse ou tout autre moyen de déshydratation poussé des boues

A noter que, dans une autre configuration, non représentée, la boue en sortie du réacteur aérobie 30 et refroidie, peut être dirigée elle aussi à partir de la conduite 41 avant la dilution 60 vers la déshydratation 50.

Une recirculation des boues aérées, sortant du réacteur 30, vers le digesteur 20 est assurée par les conduites 32, 41 et 12, selon le sens d'écoulement indiqué par des flèches. Ce trajet de recirculation comporte de manière facultative, mais avantageuse, l'échangeur 18 qui assure un refroidissement des boues sortant du réacteur aérobie 30 pour préchauffer les boues, provenant du digesteur 20 et se dirigeant vers le réacteur aérobie 30.

Un dispositif de dilution 60, branché sur la conduite 41, est prévu pour permettre d'injecter un liquide, généralement de l'eau, afin de contrôler les conditions opératoires du digesteur 20, qui nécessite une siccité relativement réduite des boues, de l'ordre de 6% de matières sèches, pour un bon fonctionnement.

Une recirculation d'une partie des centrats, c'est-à-dire de la fraction liquide, de la déshydratation 50 est prévue avec une conduite 51 reliant la sortie des centrats au mélangeur 15. Cette recirculation contribue à gérer la dilution et le traitement des matières organiques encore solubilisées.
A noter qu'en fonction de la qualité des boues à traiter, la conduite 51 peut être dimensionnée pour recirculer plutôt les boues solides qui contiennent aussi la matière organique.

La sortie de la fraction solide non recirculée du dispositif 50 de déshydratation est reliée par une conduite 52 à une unité 60 d'évacuation de la fraction solide.

La sortie de la fraction liquide de la conduite 51 débouche aussi vers une unité 70 d'évacuation des centrats qui généralement retournent en station.

L'installation est équipée des moyens de pompage, des moyens de mesure et organes de réglage (non représentés) pour assurer tous les débits, contrôles et réglages permettant la gestion du procédé et en particulier les contrôles et réglages :
- des paramètres de fonctionnement du digesteur 20 : température, charge hydraulique, charge massique, concentration, pH...
- des paramètres de fonctionnement de l'étape aérobie 30 : température, charge...
- du taux de recirculation des différents flux par les conduites 21,41, 51
- du taux de nitrates et d'ammonium dans le digesteur.

Selon la configuration mise en oeuvre dans le schéma du dessin, le procédé objet de la présente invention, permet, par l'intermédiaire du traitement aérobie, de limiter, dans l'étape de digestion anaérobie, les phénomènes de moussage dus à la présence de micro-organismes filamenteux.

Par ailleurs, ces configurations permettent d'améliorer de 1 à 20 points la siccité des boues résiduelles après traitement de déshydratation par rapport à une digestion anaérobie conventionnelle.

Le fonctionnement de l'installation est le suivant.

Les boues 1 à traiter subissent une déshydratation dans le dispositif 10 pour présenter en sortie une siccité d'au moins 20 à 30% de matières sèches. Une partie importante de l'eau des boues 1 a été éliminée de sorte que le volume de boues à traiter par le digesteur 20 est réduit et les dimensions du digesteur 20 s'en trouvent sensiblement diminuées.

Toutefois, une telle siccité est trop élevée pour un bon fonctionnement du digesteur 20. La recirculation de boues solubilisées provenant du digesteur 20, par la conduite 21, et/ou du réacteur aérobie 30, par la conduite 41, et /ou du dispositif de déshydratation finale 50, par la conduite 51, permet d'obtenir, en sortie du mélangeur 15, dans la conduite 12, une boue dont la siccité convient pour la digestion, généralement une siccité d'environ 6% de matières sèches en masse. Le taux de recirculation est choisi pour obtenir cette siccité en entrée du digesteur 20.

Les boues sont digérées et solubilisées en partie dans le digesteur 20. Une partie des boues digérées est recyclée par la conduite 21, comme déjà exposé ; l'autre partie est évacuée par la conduite 22, puis la conduite 43. Le flux est ensuite séparé entre la conduite 45, et la conduite 47. La conduite 45 dirige le flux vers le dispositif 40 de dilacération/dessablage. Le flux sort par la conduite 48 pour entrer dans le réacteur 30 et y subir un traitement aéré, de préférence thermique à environ 60-70°C. Les boues traitées dans le réacteur 30 sortent par la conduite 32 et sont dirigées par la conduite 41 vers le mélangeur 15.

La conduite 47 dirige les boues vers le dispositif 50 de déshydratation finale. Les centrats, ou fraction liquide, du dispositif 50 sont dirigés vers le mélangeur 15, tandis que la fraction solide est dirigée, au moins en partie, par la conduite 52 vers l'évacuation 60. Une fraction des centrats est évacuée du système, à partir de la conduite 51, vers l'évacuation 70. Une conduite 47b permet aussi de diriger une partie de la boue, de la conduite 41, vers la déshydratation 50.

La déshydratation assurée par le dispositif 50 permet d'éviter une concentration des nitrates produits dans le réacteur aérobie 30 et de l'ammoniac produit par la digestion. En effet, les nitrates en sortie du réacteur aérobie sont sous forme liquide. En passant dans la déshydratation 50, ils sont purgés avec les centrats évacués en 70. L'ammoniac produit par le digesteur est aussi sous forme liquide ; il peut être soit purgé directement en 47 par l'intermédiaire de la déshydratation 50 et de l'évacuation des centrats 70, soit transformé en nitrates dans l'étape aérobie 30.

Au démarrage de l'installation, le temps de séjour des boues dans le digesteur 20 est plus important qu'en régime permanent, jusqu'à ce que les boues solubilisées recyclées permettent d'obtenir en entrée du digesteur 20 la siccité souhaitée.

Grâce au procédé selon l'invention :
- les dimensions du digesteur 20 sont réduites par rapport à celles d'un digesteur de l'état de la technique ;
- le rendement de digestion est amélioré ;
- la production finale de boues est diminuée car la matière organique est davantage transformée en dioxyde de carbone et en méthane ;
- l'ammoniac et les nitrates sont purgés.

## Revendications

1. Procédé de réduction de la production de boues de stations d'épuration d'eaux usées urbaines ou industrielles, comprenant une étape de digestion anaérobie (20) mésophile ou thermophile, ou associant ces deux modes de fonctionnement, d'un flux de boues à traiter (1), et au moins une étape de traitement aérobie (30) de solubilisation biologique, **caractérisé en ce qu'**il comporte en amont de l'étape de digestion anaérobie :
- une étape de déshydratation (10) des boues à traiter,
- suivie d'une étape de mélange (15) des boues déshydratées avec une fraction recirculée de boues plus liquides provenant d'un recyclage de la digestion (20), et/ou de l'étape de traitement aérobie (30), et/ou de centrats et/ou de boues provenant d'une déshydratation finale (50) des boues traitées, le taux de recirculation étant choisi pour que le mélange présente une siccité adaptée à la digestion, ce mélange étant ensuite dirigé vers la digestion,
l'étape de traitement aérobie (30) de solubilisation biologique étant située en aval de la digestion anaérobie.

2. Procédé selon la revendication 1, **caractérisé en ce que** la siccité des boues en sortie de la première étape de déshydratation (10) est comprise entre 20 et 30% de matières sèches en masse, et le taux de recirculation est choisi pour que la siccité des boues en sortie du mélange (15) et à l'entrée de la digestion soit inférieure à 10% de matières sèches en masse, de préférence d'environ 6%.

3. Procédé selon l'une quelconque des revendications précédentes, **caractérisé en ce que** l'étape aérobie (30) de solubilisation biologique est située sur une boucle de recirculation de l'étape de digestion anaérobie (20).

4. Procédé selon la revendication 1, **caractérisé en ce qu'**il comporte une étape finale de déshydratation (50), en particulier par centrifugation.

5. Procédé selon la revendication 1, **caractérisé en ce qu'**il comporte une mesure de nitrates en entrée de la digestion (20), une mesure d'ammonium et de pH en sortie de la digestion (20), et une régulation de la recirculation à partir de ces mesures.

6. Procédé selon l'une quelconque des revendications précédentes, **caractérisé en ce qu'**il comporte une étape de dilacération/dessablage (40) de la boue permettant de diminuer l'ensablement du digesteur, l'abrasion des composants et l'agglomération de filasses dans le circuit.

7. Procédé selon l'une quelconque des revendications précédentes, **caractérisé en ce qu'**une partie du biogaz produit lors de la digestion anaérobie des boues est utilisée comme source d'énergie pour chauffer ou maintenir en température un réacteur (30) dans lequel s'effectue l'étape aérobie de solubilisation biologique.

8. Procédé selon l'une quelconque des revendications précédentes, **caractérisé en ce qu'**un échange de chaleur (18) est effectué entre des boues sortant de l'étape aérobie (30) de solubilisation biologique et des boues sortant de l'étape de digestion anaérobie (20) et qui sont dirigées vers l'étape aérobie, pour que le procédé soit énergétiquement auto-équilibré et que la consommation énergétique soit réduite.

9. Procédé selon la revendication 8, **caractérisé en ce que** l'échange de chaleur est du type indirect boues/eau, eau/boues, l'eau servant de fluide caloporteur pour l'échange entre les deux boues.

10. Procédé selon l'une quelconque des revendications précédentes, **caractérisé en ce qu'**une dilution (60) est prévue sur une boucle de recirculation reliée à la sortie de l'étape aérobie, pour permettre de piloter les conditions de fonctionnement de la digestion.

11. Procédé selon l'une quelconque des revendications précédentes, **caractérisé en ce que** des flux de retour (41, 51) provenant de l'étape aérobie, ou d'une déshydratation finale sont pilotés, en particulier en fonction des conditions de fonctionnement de la digestion et de la production de biogaz et en fonction de la qualité du résidu solide (taux de matières volatiles, taux d'azote).

12. Installation pour la mise en oeuvre d'un procédé selon la revendication 1, pour la réduction de la production de boues de stations d'épuration d'eaux usées urbaines ou industrielles, comprenant un digesteur anaérobie (20) de type mésophile ou thermophile des boues, ou associant ces deux modes de fonctionnement, d'un flux de boues à traiter, et au moins un réacteur de traitement aérobie (30) de solubilisation biologique des boues, **caractérisée en ce qu'**elle comporte en amont du digesteur anaérobie (20) :
- un dispositif de déshydratation (10) des boues à traiter,
- suivi d'un mélangeur (15) des boues déshydratées avec une fraction recirculée de boues plus liquides délivrées par une conduite de recyclage (21) provenant du digesteur (20), et/ou par une conduite (41) provenant du réacteur de traitement aérobie (30), et/ou par une conduite (51) de centrats et/ou d'une fraction de boues solides provenant d'une déshydratation finale (50) des boues traitées, le taux de recirculation étant choisi pour que le mélange présente une siccité adaptée à la digestion, et que les concentrations en nitrates soient adaptées à la digestion, ce mélange étant ensuite dirigé vers la digestion,
le réacteur de traitement aérobie (30) étant situé en aval du digesteur anaérobie (20).

13. Installation selon la revendication 12, **caractérisée en ce que** le réacteur de traitement aérobie (30) est situé sur une boucle de recirculation (22,43,45,48,32,41,12) du digesteur anaérobie (20).

14. Installation selon l'une quelconque des revendications 12 ou 13, **caractérisée en ce qu'**elle comporte un dispositif de déshydratation finale (50), en particulier une centrifugeuse.

15. Installation selon l'une quelconque des revendications 12 à 14, **caractérisée en ce qu'**elle comporte un dispositif (40) de dilacération/dessablage de la boue permettant de diminuer l'ensablement du digesteur, l'abrasion des composants et l'agglomération de filasses dans le circuit.

16. Installation selon l'une quelconque des revendications 12 à 15, **caractérisée en ce qu'**elle comporte un dispositif de chauffage (40) du réacteur aérobie (30), alimenté (23) par une partie du biogaz produit par le digesteur anaérobie (20) des boues.

17. Installation selon l'une quelconque des revendications 12 à 16, **caractérisée en ce qu'**elle comporte un échangeur de chaleur (18) entre des boues sortant du réacteur aérobie (30) et des boues sortant du digesteur anaérobie (20) et qui sont dirigées vers le réacteur aérobie (30).

18. Installation selon l'une quelconque des revendications 12 à 17, **caractérisée en ce qu'**elle comporte un dispositif de dilution (60) prévu sur une boucle de recirculation provenant de la sortie du réacteur aérobie, pour permettre de piloter les conditions de fonctionnement de la digestion.

19. Installation selon l'une quelconque des revendications 12 à 18, **caractérisée en ce qu'**elle comporte une sonde de nitrates sur l'entrée (12) du digesteur et une sonde d'ammonium et de pH sur la sortie (22) du digesteur.

## Patentansprüche

1. Verfahren zur Verringerung der Erzeugung von Schlämmen durch Anlagen zur Reinigung von städtischen oder industriellen Abwässern, mit einem Schritt der mesophilen oder thermophilen anaeroben Gärung (20) eines zu behandelnden Schlammstroms (1), oder mit der Kombination der beiden Betriebsmodi, und mit mindestens einem aeroben Behandlungsschritt (30) der biologischen Solubilisierung, **dadurch gekennzeichnet, dass** es vor dem anaeroben Gärungsschritt aufweist:
- einen Schritt (10) zur Dehydratation der zu behandelnden Schlämme,
- gefolgt von einem Schritt (15) des Mischens der dehydrierten Schlämme mit einem zurückgeführten Anteil von flüssigeren Schlämmen, die aus einer Wiederverwertung der Gärung (10) und/oder aus dem Schritt (30) zur aeroben Behandlung stammen, und/oder mit Rückläufen und/oder aus einer finalen Dehydratation (50) der behandelten Schlämme stammendem Schlämmen, wobei das Rücklaufverhältnis derart gewählt ist, dass die Mischung eine für die Gärung geeignete Trockenheit aufweist, wobei diese Mischung anschließend der Gärung zugeführt wird,
wobei der aerobe Behandlungsschritt (30) der biologischen Solubilisierung hinter der anaeroben Gärung angeordnet ist.

2. Verfahren nach Anspruch 1, **dadurch gekennzeichnet, dass** die Trockenheit der Schlämme am Ausgang des ersten Dehydratationsschritts (10) zwischen 20 und 30 Massenprozent bezogen auf die Trockenmasse beträgt, und das Rücklaufverhältnis derart gewählt ist, dass die Trockenheit der Schlämme am Ausgang des Mischens (15) und am Eingang der Gärung geringer als 10 Massenprozent bezogen auf die Trockenmasse ist, vorzugsweise ungefähr 6% beträgt.

3. Verfahren nach einem der vorhergehenden Ansprüche, **dadurch gekennzeichnet, dass** der aerobe Schritt (30) der biologischen Solubilisierung in einem Rückführkreislauf des anaeroben Gärungsschritts (20) angeordnet ist.

4. Verfahren nach Anspruch 1, **dadurch gekennzeichnet, dass** es einen finalen Schritt (50) der Dehydratation, insbesondere durch Zentrifugieren, aufweist.

5. Verfahren nach Anspruch 1, **dadurch gekennzeichnet, dass** es eine Messung der Nitrate am Eingang der Gärung (20), eine Ammonium- und eine pH-Messung am Ausgang der Gärung (20) und eine Regelung der Rückführung auf der Basis dieser Messungen aufweist.

6. Verfahren nach einem der vorhergehenden Ansprüche, **dadurch gekennzeichnet, dass** es einen Schritt zur Zerkleinerung und Entsandung (40) des Schlamms aufweist, welche eine Verringerung der Versandung des Faulbehälters, des Abriebs von Bauteilen und der Ansammlung von Faserstoffen in dem Kreislauf ermöglicht.

7. Verfahren nach einem der vorhergehenden Ansprüche, **dadurch gekennzeichnet, dass** ein Teil des bei der anaeroben Vergärung der Schlämme erzeugten Biogases als Energiequelle zum Heizen oder zum Aufrechterhalten der Temperatur eines Reaktors (30) verwendet wird, in welchem der aerobe Schritt der biologischen Solubilisierung stattfindet.

8. Verfahren nach einem der vorhergehenden Ansprüche, **dadurch gekennzeichnet, dass** ein Wärmetausch (18) zwischen den aus dem aeroben Schritt (30) der biologischen Solubilisierung austretenden Schlämmen und den aus dem Schritt der anaeroben Gärung (20) austretenden und zu dem aeroben Schritt geleiteten Schlämmen erfolgt, so dass das Verfahren energetisch selbst-regulierend ist und der Energieverbrauch verringert ist.

9. Verfahren nach Anspruch 8, **dadurch gekennzeichnet, dass** der Wärmtausch vom indirekten Typ zwischen Schlämmen und Wasser, Wasser und Schlämmen ist, wobei das Wasser als Wärmeübertragungsflüssigkeit für den Austausch zwischen den beiden Schlämmen dient.

10. Verfahren nach einem der vorhergehenden Ansprüche, **dadurch gekennzeichnet, dass** eine Verdünnung (60) in einem Rückführkreislauf vorgesehen ist, der mit dem Ausgang des aeroben Schritts verbunden ist, um das Steuern der Betriebsbedingungen der Gärung zu ermöglichen.

11. Verfahren nach einem der vorhergehenden Ansprüche, **dadurch gekennzeichnet, dass** von dem aeroben Schritt oder einer finalen Dehydratation kommende Rückflüsse (41, 51) gesteuert werden, insbesondere in Abhängigkeit von den Betriebsbedingungen der Gärung und der Biogaserzeugung und in Abhängigkeit von der Qualität der festen Rückstände (Anteil der flüchtigen Stoffe, Stickstoffanteil).

12. Anlage zur Durchführung eines Verfahrens nach Anspruch 1, zur Verringerung der Erzeugung von Schlämmen durch Anlagen zur Reinigung von städtischen oder industriellen Abwässern, mit einem anaeroben Schlamm-Faulbehälter (20) vom mesophilen oder thermophilen Typ, oder mit der Kombination der beiden Betriebsmodi, für einen zu behandelnden Schlammstrom, und mit mindestens einem aeroben Behandlungsreaktor (30) zur biologischen Solubilisierung der Schlämme, **dadurch gekennzeichnet, dass** sie stromaufwärts des anaeroben Faulbehälters (20) aufweist:
- eine Vorrichtung (10) zur Dehydratation der zu behandelnden Schlämme,
- gefolgt von einem Mischer (15) der dehydrierten Schlämme mit einem zurückgeführten Anteil von flüssigeren Schlämmen, die über eine Wiederverwertungsleitung (21) des Faulbehälters (20) und/oder über eine von dem aeroben Behandlungsreaktor (30) kommende Leitung (41), und/oder über eine Rücklauf-Leitung (51) zugeführt werden, und/oder mit einem Anteil von festen Schlämmen, die aus einer finalen Dehydratation (50) der behandelten Schlämme, wobei das Rücklaufverhältnis derart gewählt ist, dass die Mischung eine Trockenheit aufweist, die für die Gärung geeignet ist, und dass die Nitratkonzentrationen für die Gärung geeignet sind, wobei diese Mischung anschließend der Gärung zugeführt wird und der anaerobte Behandlungsreaktor (30) stromabwärts des anaeroben Faulbehälters (20) angeordnet ist.

13. Anlage nach Anspruch 12, **dadurch gekennzeichnet, dass** der aerobe Behandlungsreaktor (30) in einem Rückführkreislauf (22, 43, 45, 48, 32, 41, 12) des anaeroben Faulbehälters (20) angeordnet ist.

14. Anlage nach einem der Ansprüche 12 oder 13, **dadurch gekennzeichnet, dass** sie eine Vorrichtung (50) zur finalen Dehydratation, insbesondere eine Zentrifuge, aufweist.

15. Anlage nach einem der Ansprüche 12 bis 14, **dadurch gekennzeichnet, dass** sie eine Vorrichtung (40) zur Zerkleinerung/Entsandung des Schlamms aufweist, welche eine Verringerung der Versandung des Faulbehälters, des Abriebs von Bauteilen und der Ansammlung von Faserstoffen in dem Kreislauf ermöglicht.

16. Anlage nach einem der Ansprüche 12 bis 15, **dadurch gekennzeichnet, dass** sie eine Vorrichtung (40) zur Erwärmung des aeroben Reaktors (30) aufweist, die durch einen Teil des von dem anaeroben Schlamm-Faulbehälter (20) erzeugten Biogases gespeist (23) ist.

17. Anlage nach einem der Ansprüche 12 bis 16, **dadurch gekennzeichnet, dass** sie einen Wärmetauscher (18) zwischen den aus dem aeroben Reaktor (30) kommenden Schlämmen und den aus dem anaeroben Faulbehälter (20) kommenden und zu dem aeroben Faulbehälter (30) geleiteten Schlämmen aufweist.

18. Anlage nach einem der Ansprüche 12 bis 17, **dadurch gekennzeichnet, dass** sie eine Verdünnungsvorrichtung (60) aufweist, die in einem von dem Ausgang des aeroben Reaktors kommenden Rückführkreislauf vorgesehen ist, um das Steuern der Betriebsbedingungen der Gärung zu ermöglichen.

19. Anlage nach einem der Ansprüche 12 bis 18, **dadurch gekennzeichnet, dass** sie eine Nitratsonde am Eingang (12) des Faulbehälters und eine Ammonium- und pH-Sonde am Auslass (22) des Faulbehälters aufweist.

## Claims

1. A process for reducing the production of sludge by municipal or industrial wastewater purification plants, comprising a step of mesophilic or thermophilic anaerobic digestion (20), or digestion which associates these two operating modes, of a flow of sludge to be treated (1), and at least one aerobic treatment step (30) of biological solubilization, **characterized in that** it comprises, upstream from the anaerobic digestion step:
- a step of dehydration (10) of the sludge to be treated;
- followed by a step of mixing (15) dehydrated sludge with a recirculated fraction of sludge that is more liquid obtained from recycling of the digestion (20), and/or from the aerobic treatment step (30), and/or from centrates and/or from sludge originating from final dehydration (50) of the treated sludge, the level of recirculation being selected such that the mixture has dryness suitable for the digestion, this mixture then being directed towards the digestion,
the aerobic treatment step (30) of biological solubilization being situated downstream from the anaerobic digestion.

2. The process as claimed in claim 1, **characterized in that** the dryness of the sludge at the output of the first dehydration step (10) is between 20 and 30% by weight of dry materials, and the level of recirculation is selected such that the dryness of the sludge at the output of the mixture (15) and at the input of the digestion is less than 10% by weight of dry materials, and preferably approximately 6%.

3. The process as claimed in any one of the preceding claims, **characterized in that** the aerobic step (30) of biological solubilization is situated on a recirculation loop of the anaerobic digestion step (20).

4. The process as claimed in claim 1, **characterized in that** it comprises a final step of dehydration (50), in particular by centrifuging.

5. The process as claimed in claim 1, **characterized in that** it comprises a measurement of nitrates at the input of the digestion (20), a measurement of ammonium and pH at the output of the digestion (20), and regulation of the recirculation on the basis of these measurements.

6. The process as claimed in any one of the preceding claims, **characterized in that** it comprises a step of dilaceration/de-sanding (40) of the sludge, thus making it possible to decrease the silting up of the digester, the abrasion of the components, and the agglomeration of fibers in the circuit.

7. The process as claimed in any one of the preceding claims, **characterized in that** part of the biogas produced during the anaerobic digestion of the sludge is used as a source of energy to heat or maintain the temperature of a reactor (30) in which the aerobic step of biological solubilization is carried out.

8. The process as claimed in any one of the preceding claims, **characterized in that** heat exchange (18) takes place between the sludge which is discharged from the aerobic step (30) of biological solubilization and the sludge which is discharged from the anaerobic digestion step (20), and is directed to the aerobic step, in order for the process to be energetically self-balanced, and for the energy consumption to be reduced.

9. The process as claimed in claim 8, **characterized in that** the exchange of heat is of the indirect sludge/water, water/sludge type, with the water acting as a heat-exchanging fluid for the exchange between the two sludges.

10. The process as claimed in any one of the preceding claims, **characterized in that** dilution (60) is provided on a recirculation loop which is connected to the aerobic step output, in order to make it possible to control the operating conditions of the digestion.

11. The process as claimed in any one of the preceding claims, **characterized in that** return flows (41, 51) obtained from the aerobic step or from final dehydration are controlled, in particular according to the operating conditions of the digestion and of the production of biogas, and according to the quality of the solid residue (levels of volatile materials and nitrogen).

12. An installation for implementation of a process as claimed in claim 1, for reducing the production of sludge by municipal or industrial wastewater purification plants, comprising an anaerobic sludge digester (20) of the mesophilic or thermophilic type, or a digester which associates these two operating modes, of a flow of sludge to be treated, and at least one reactor (30) for aerobic treatment of biological solubilization of the sludge, **characterized in that** it comprises, upstream from the anaerobic digester (20):
- a device (10) for dehydration of the sludge to be treated;
- followed by a mixer (15) to mix dehydrated sludge with a recirculated fraction of sludge that is more liquid supplied by a recycling duct (21) originating from the digester (20), and/or by a duct (41) originating from the aerobic treatment reactor (30), and/or by a centrates duct (51), and/or by a fraction of the solid sludge originating from final dehydration (50) of the sludge treated, the level of recirculation being selected such that the mixture has dryness suitable for the digestion, and that the concentrations of nitrates are suitable for the digestion, this mixture then being directed towards the digestion, the aerobic treatment reactor (30) being situated downstream from the anaerobic digester (20).

13. The installation as claimed in claim 12, **characterized in that** the aerobic treatment reactor (30) is situated on a recirculation loop (22,43,45,48,32,41,12) of the anaerobic digester (20).

14. The installation as claimed in any one of claims 12 or 13, **characterized in that** it comprises a final dehydration device (50), in particular a centrifuge.

15. The installation as claimed in any one of claims 12 to 14, **characterized in that** it comprises a device (40) for dilaceration/de-sanding of the sludge, thus making it possible to decrease the silting up of the digester, the abrasion of the components, and the agglomeration of fibers in the circuit.

16. The installation as claimed in any one of claims 12 to 15, **characterized in that** it comprises a device (40) for heating of the aerobic reactor (30), supplied (23) by part of the biogas produced by the anaerobic digester (20) of the sludge.

17. The installation as claimed in any one of claims 12 to 16, **characterized in that** it comprises a heat exchanger (18) between sludge which is discharged from the aerobic reactor (30) and sludge which is discharged from the anaerobic digester (20) and is directed to the aerobic reactor (30).

18. The installation as claimed in any one of claims 12 to 17, **characterized in that** it comprises a dilution device (60) which is provided on a recirculation loop originating from the output of the aerobic reactor, in order to make it possible to control the operating conditions of the digestion.

19. The installation as claimed in any one of claims 12 to 18, **characterized in that** it comprises a sensor for nitrates at the input (12) of the digester and a sensor for ammonium and pH at the output (22) of the digester.
